(19) Europäisches Patentamt / European Patent Office / Office européen des brevets

(11) **EP 3 187 110 A1**

(12) **EUROPEAN PATENT APPLICATION**

(43) Date of publication:
**05.07.2017 Bulletin 2017/27**

(51) Int Cl.:
*A61B 5/0476* (2006.01)   *A61B 5/0478* (2006.01)
*A61B 5/00* (2006.01)

(21) Application number: **16207160.9**

(22) Date of filing: **28.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.12.2015 EP 15203082**

(71) Applicant: **squipe GmbH**
**8044 Zürich (CH)**

(72) Inventor: **HILTY, Lea**
**8044 Zürich (CH)**

(74) Representative: **Liebetanz, Michael**
**Isler & Pedrazzini AG**
**Giesshübelstrasse 45**
**Postfach 1772**
**8027 Zürich (CH)**

(54) **APPARATUS FOR DETECTING AND PROVIDING BRAIN SIGNALS BY USE OF ELECTROENCEPHALOGRAPHY**

(57) Apparatus for monitoring a brain signal, especially indicative of muscle fatigue, comprises at least three sensors sensing neuronal activity, wherein one sensor (11) comprises a reference electrode and the other sensors (15, 16) comprise sensing electrodes, wherein the reference sensor (11) serves as a common reference for the other sensors (15, 16). The reference sensor (11) is placed within the quadrangle defined by the four positions [F5-F1-C1-C5] or on a corridor of 10 millimetres width along the line between Fpz to Oz, the first sensing sensor is placed within the quadrangle [C1-C2-P2-P1], and the second sensing sensor is placed within the quadrangle [FT7-FC3-CP3-TP7], with the proviso that the reference electrode is not positioned on the same position as is one of the two sensing sensors and wherein the positions of the sensors delimiting the quadrangles are defined according to the 10-10 Electrode Placement System of the American Clinical Neurophysiology Society.

**Fig. 4**

EP 3 187 110 A1

**Description**

TECHNICAL FIELD

The present invention relates to an apparatus for detecting and providing brain signals by use of electroencephalography (EEG).

PRIOR ART

In the prior art, many devices are related to the technical field of handling of biosignals, and more particularly to measure and display brain signals by use of EEG. The EEG method enables to measure electrophysiological activity of the brain by recording from electrodes placed on the scalp. EEG biofeedback devices, also known as neurofeedback devices, provide real-time feedback on certain electrophysiological activity signals enabling users to learn e.g. to control or manipulate their own electrophysiological activity. As such, neurofeedback devices and methods have been used as an aid e.g. to learn to establish or dissolve various states of attention and consciousness (US4031883A), to enhance perception and cognition (WO2010147599A1) and to control arousal level and the degree of distractibility (EP0381090A2). In clinical settings, neurofeedback has been used e.g. to enhance attention in attention-deficit/hyperactivity disorder (ADHD) (US2002120208 A1) and as treatment method for chronic fatigue syndrome promoting patients to decrease slow wave activity (US5267570A).

In sports, EEG biofeedback applications have been applied in order to improve the mental state for performing activities like playing a shot in golf (WO2013040642A1) and to focus attention and improve concentration in shooting (KR20030021585A).

Taken together, EEG biofeedback has a wide variety of applications and may provide cognitive, emotional and clinical benefits by teaching the user to manage to achieve a certain mental condition. However, so far, it has not been possible to use EEG biofeedback to measure and visualize a brain signal indicative of muscle fatigue. Research using laboratory methods with offline analysis could demonstrate that muscle fatigue is comprised of peripheral (intramuscular) and central (residing in the central nervous system) processes (e.g. Bigland-Ritchie et al., Clin Sci Mol Med 1978;54:609-14; Taylor et al., Clin Exp Pharmacol Physiol 2006;33:400-405). Whereas, to date, different peripheral processes (e.g. lactate measurement) related to muscle fatigue can be assessed easily, it has not been possible yet to measure and display central processes in the brain indicative of muscle fatigue by use of a portable system applicable by laymen during sports activities.

Prior art has demonstrated the capability to monitor muscle fatigue related brain activity using a stationary EEG system with wired connection to a PC, making use of 128 channels and relying on post-hoc analysis methods (Hilty et al., Eur J Neurosci 2011;34:2035-2042). This kind of system is not fit to be used during outdoor activities and the amount of data acquired with this kind of system cannot be processed with current technology in real-time, rendering its application as exercise monitoring device practically impossible.

Few articles exist investigating EEG activity during endurance activities and they use different methodologies.

One research group around Nielsen used only two electrodes prefrontal locations (F3 and F4) and derived measures composed of power spectra from several frequency bands, published by P. Rasmussen, H. Stie, L. Nybo, B. Nielsen, as "Heat induced fatigue and changes of the EEG is not related to reduced perfusion of the brain during prolonged exercise in humans", in Journal of Thermal Biology, Volume 29, Issues 7-8, October-December 2004, Pages 731-737, ISSN 0306-4565, http://dx.doi.org/10.1016/j.jtherbio.2004.08.047.

Another group from Penn State University checked from 30 standard positions, which pick up activity changes during the course of increasing muscle fatigue. They describe changes in movement related potentials picked up from electrodes at Cz, FCz, and C3. However, such potentials can't be picked up during continuous movements, but only be related to initiation of movements, which renders this research inapplicable when designing an apparatus to measure the cortical contribution to muscle fatigue during endurance sports. J Johnston, M Rearick, S Slobounov published "Movement-related cortical potentials associated with progressive muscle fatigue in a grasping task", Clinical Neurophysiology, Volume 112, Issue 1, January 2001, Pages 68-77, ISSN 1388-2457, http://dx.doi.org/!0.1016/S1388-2457(00)00452-1.

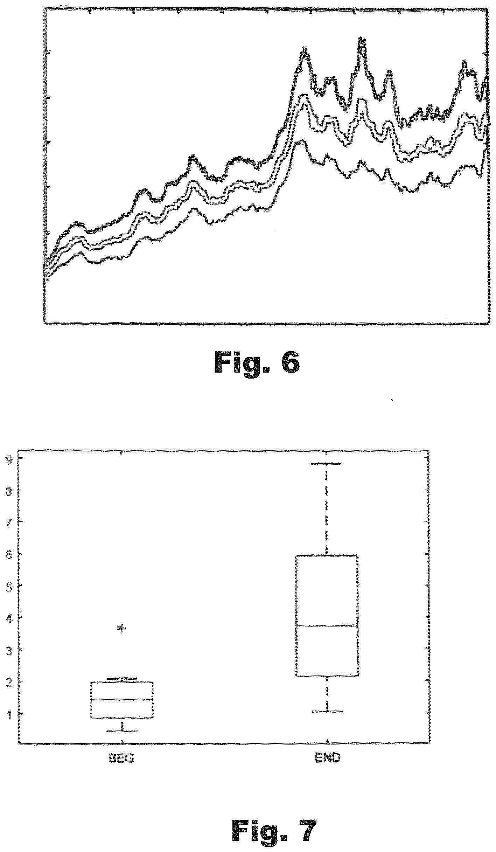

The same problem applies to work by Liu et al. 2006: They have used a repetitive fatiguing handgrip task and show strongest EEG changes related to fatigue in Pz, Cz, C3 and C4 (Fig 7 from Liu et al. 2006). Here, again, due to locking of brain signals to events in time no clear inference can be drawn for a situation where a continuous process would be evaluated, as during endurance sports. The publication is Liu, J. Z., Yao, B., Siemionow, V., Sahgal, V., Wang, X., Sun, J., & Yue, G. H. (2005) "Fatigue induces greater brain signal reduction during sustained than preparation phase of maximal voluntary contraction" in Brain research, 1057(1), 113-126.

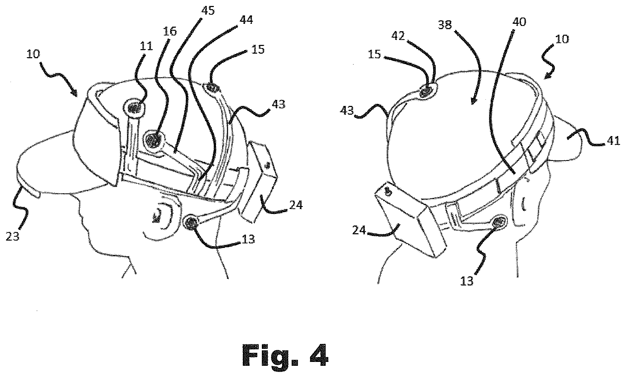

Jain et al. (2013) describe electrode locations relevant to pick up EEG signals during continuous locomotor activity (active vs. passive pedalling) without special focus on muscle fatigue. This information might be relevant to select electrode positions bearing relevant information related to muscle activation. Fig. 4 from Jain et al. (2013) shows that Cz (negatively correlated with electromyographic activity) or C5 (positively correlated with electromyographic activity) could be relevant. More interesting is their Fig. 6 showing differences between active and passive leg movements, which isolates a component of voluntary locomotor activity and thus may be closer to the situation during exhaustive exercise. The figure shows that possible electrode positions to record brain activity related to voluntary leg motor control are located around Fpz, C3, C4; Cz; CP3, CP4; mainly, a power decrease due to voluntary locomotor activity (blue) can be found. Only in lower frequency bands (6-10Hz), a few locations (around CP4) show increased power due to active leg movements. The article from Jain, S., Gourab, K., Schindler-Ivens, S., & Schmit, B. D. (2013) is published as "EEG during pedalling: evidence for cortical control of locomotor tasks". Clinical Neurophysiology, 124(2), 379-390.

Some further work exists, investigating hand motor activity and thus exclusively focussing on electrodes overlying the primary hand motor area, ignoring other regions as done by A. A. Abdul-latif, I. Cosic, D. K. Kumar, B. Polus and C. Da Costa with "Power changes of EEG signals associated with muscle fatigue: the root mean square analysis of EEG bands," Intelligent Sensors, Sensor Networks and Information Processing Conference, 2004. Proceedings of the 2004, 2004, pp. 531-534. This disclosure leads to the selection of only one electrode site similar to a site revealed in the current specification. However, this kind of work cannot be used as a model for electrode placement in the present case, where it is an aim to identify an EEG signal indicative of muscle fatigue during whole body endurance exercise rather than during arm or hand movements.

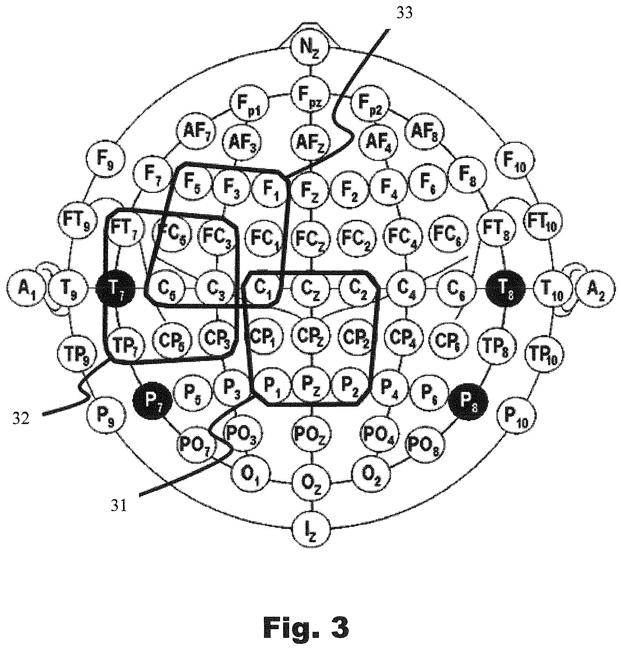

A publication touching the subject matter of surface EEG electrodes picking up insular activity in the field of emotion processing is Ponz, A., Montant, M., Liegeois-Chauvel, C., Silva, C., Braun, M., Jacobs, A. M., & Ziegler, J. C. (2014) in "Emotion processing relates to in words: a test of the neural re-use hypothesis using surface and intracranial EEG" in Social Cognitive and Affective Neuroscience, nst034. Fig. 3B from Ponz et al. (2014) shows current density distributions on the scalp surface. This EEG activity has in a second step been localized to originate from the anterior insular cortex (Fig. 4 from Ponz et al. 2014). Notably, the surface distribution shows prominent EEG activity at central locations (C1, C3, FC1), implying that these locations could be the regions most strongly influenced by insular activity.

Thus, from literature research one could identify prefrontal (F3, F4), frontal (FCz, FC1), central (Cz, C1, C3, C4, C5) or posterior (CP3, CP4, Pz) electrode positions possibly relevant to pick up signals indicative of muscle fatigue. No EEG-literature gives rise to the notion that electrodes over temporal regions might be relevant. Previous work from the inventors in Hilty, L., Langer, N., Pascual-Marqui, R., Boutellier, U. and Lutz, K. (2011), "Fatigue-induced increase in intracortical communication between mid/anterior insular and motor cortex during cycling exercise" from European Journal of Neuroscience, 34: 2035-2042. doi:10.1111/j.1460-9568.2011.07909. does not disclose which electrodes pick up the relevant information being attributed to the 3D regions "insular cortex" and "primary motor cortex" formed by means of sLORETA source modelling.

SUMMARY OF THE INVENTION

Based on this prior art already evaluated in the above paragraph it is an object of the invention to provide improvements over this prior art creating a portable device being able to monitor muscle fatigue as a lightweight and portable system working under conditions of outdoor activity.

The invention relies inter alia on the insight that a reduced set of channels is sufficient to determine and obtain the muscle fatigue information. Whereas prior art (Hilty et al., Eur J Neurosci 2011;34:2035-2042) has relied on highly complex recording and analysis methods, the invention now identifies a small number of necessary channels and changed the processing steps, thus reducing the required energy, computational power, and hardware of the device. The required reduction in complexity has been achieved by the following steps: originally the method required calculation of a current density distribution over a 3D head model space and selection of two 3D regions from this space. Within these regions, average current density was determined every 2 ms to determine the 3D current density distribution, the data of 108 channels were used, semi-automatically scrutinized for and cleaned from artefacts, and filtered. The resulting time series of current density oscillations were cut in sections of one minute length at specific times and within these epochs, lagged phase synchronization was determined between the two time series.

The concept behind the measurement performed by the apparatus disclosed in the current application is the measurement of communication between two regions relevant for the brains contribution to muscle fatigue: the insular cortex and the motor cortex.

Some of these operations prevent the process from being performed online. E.g., this way of removing artefacts can only be done post hoc. Furthermore, instead of selecting specific time windows, in a mobile real-time device, the process has to allow for continuous calculation of a result over a short period of a few seconds in the past. Most importantly, the calculation has to be made less complex such that with current technology, it is possible to implement a suitable algorithm into a low-energy digital signal processor.

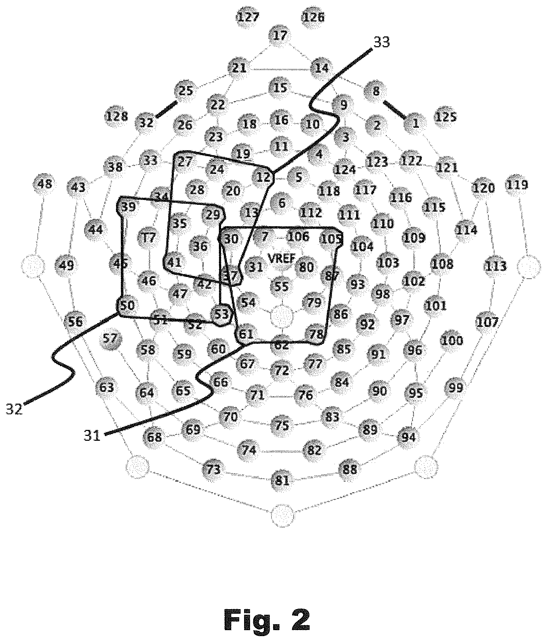

Thus, the first reduction in complexity which the current device has over the prior art is the usage of two electric currents derived from bipolar recordings on the surface of the scalp instead of current densities in a 3D space. To achieve this, recording positions were found bearing the essential information with a sufficient signal to noise ratio. The identification of these positions is not trivial, since the regions from which the signal has to be generated are 1) not on the surface of the cerebral cortex, and 2) the neurons acting as signal sources, are not necessarily oriented apical to the surface of the scalp, making it hard to predict how the electromagnetic fields of these sources will propagate. The result gives the electrode positions described in Figure 2.

The current invention, thus, provides the first portable EEG biofeedback device and method measuring and displaying in real time a muscle fatigue related signal in the brain.

An apparatus for monitoring a brain signal, especially indicative of muscle fatigue, comprises at least three sensors sensing neuronal activity, wherein one sensor comprises a reference electrode and the other sensors comprise sensing electrodes, wherein the sensor with the reference electrode serves as a common reference for the other sensors. Furthermore, a ground electrode is provided at a mastoid, an earlobe or another place of the head of the user. The reference sensor is placed within the quadrangle defined by the four positions [F5-F1-C1-C5] or on the midline of the scalp as defined by a corridor of up to 15 millimetres width between Fpz to Oz. The placement can be on one of these points or in between provided that it's center is positioned inside the predetermined area, which has a width of less than 15 millimetre, preferably less than about 10 millimetres. In other words, the reference sensor is placed on the midline of the scalp, as defined by a corridor of up to 15 or preferably up to 10 millimetres width between Fpz to Oz. The first sensing sensor is placed within the quadrangle defined by the four positions [C1-C2-P2-P1] and the second sensing sensor is placed within the quadrangle defined by the four positions [FT7-FC3-CP3-TP7]. This would allow placement of the reference sensor at the same place. This is not intended. Therefore an apparatus according to the invention follows the proviso that the reference electrode is not positioned on the same position as is one of the two sensing sensors. Not being at the same position means according to a preferred embodiment not closer than 10 millimetres. This distance is measured between the borders of the respective electrodes. The positions of the sensors as mentioned above, delimiting the quadrangles, are defined according to the 10-10 Electrode Placement System (American Clinical Neurophysiology Society, Guideline 5: Guidelines for Standard Electrode Position Nomenclature, 2006). Of course the above positions are related to a specific side of the scalp. In such an alternative, [F2-F6-C6-C2] or the midline of the scalp as defined by a corridor of up to 15 millimetres width between FPz to Oz provides the positioning area for the reference electrode, while the first sensing sensor is placed within the quadrangle defined by the four positions [C1-C2-P2-P1], and the second sensing sensor is placed within the quadrangle defined by the four positions [FC4-FT8-TP8-CP4].

According to the present invention, a portable EEG biofeedback apparatus and method for real-time monitoring of a brain signal indicative of the degree of central muscle fatigue comprises at least two active electrodes and at least one reference electrode to be placed on the head of a user or athlete to detect the bioelectric signal. Additionally, at least one ground electrode is located on a mastoid, an ear lobe etc.. A preamplifier amplifies bioelectric sensor signals and amplified data is transmitted to a signal processor. Preferably, the signal processor is a digital signal processor of a computer. The signal processor performs a multitude of transformations on the amplified bioelectric signal to determine how the brain regions generating the signal interact and quantifies this interaction on a ratio scale. These transformations include 1) Discrete Fourier transform of both signal time series; 2) Normalization of the Fourier transform to obtain phase information, insensitive to amplitude information; 3) Calculation of the complex valued cross spectra between the two normalized Fourier transforms; 4) Separation of the lagged components of phase synchronization from the instantaneous components by using the following formula derived in Pascual-Marqui (RD Pascual-Marqui: Instantaneous and lagged measurements of linear and nonlinear dependence between groups of multivariate time series: frequency decomposition. arXiv:0711. 1455 [stat.ME], 2007-November-09, http://arxiv.org/abs/0711.1455):

$$PS_{lagged} = \sqrt{\frac{[\mathrm{Im}(S_{\check{x}\check{y}\omega})]^2}{1-[\mathrm{Re}(S_{\check{x}\check{y}\omega})]^2}}$$ where $S_{\check{x}\check{y}\omega}$ denotes the cross spectra between the two normalized Fourier transforms $\check{x}$ and $\check{y}$ at frequency $\omega$, Im denotes the imaginary part, and Re denotes the real part. Elimination of the instantaneous components is done in order to avoid contamination of the result by non-physiological effects like volume conduction or blurring. See Pascual-Marqui (RD Pascual-Marqui: Instantaneous and lagged measurements of linear and nonlinear dependence between groups of multivariate time series: frequency decomposition. arXiv:0711. 1455 [stat.ME], 2007-November-09, http://arxiv.org/abs/0711.1455) for a detailed description of the approach. The resulting value is indicative of supra-spinal muscle fatigue.

An important aspect of the present invention is the capability of the device to pick up brain activity directly or indirectly related to the development of muscle fatigue with minimal required hardware, making the system useful during sports activities. Therefore, the number of signals recorded as well as the sampling rate is kept as low as possible, requiring elaborated information about electrode placement and relevant signal properties. The present publication reveals this information.

A desirable feature is the possibility to store unprocessed EEG data from at least two channels for offline analysis and for quality control (storage of data). In a specific embodiment, 8GB NAND-Flash can be used for data storage. The reduced amount of such unprocessed EEG data is a further favorable effect of the invention.

Another desirable feature is the use of a data format, which allows for easy data transfer via internet (internet community / data upload) onto a dedicated server. In a specific embodiment, HDF5 (www.hdfgroup.org) can be used as data format.

A further feature of the invention is the possibility to inform the athlete wearing the system about the specific fatigue parameter determined, in real time, while the athlete is engaging in sports activities. In a specific embodiment, the parameter determined can be transformed to a color scale and displayed via RGB-LED on a display, which can be consulted by the athlete. In another embodiment, the parameter can be normalized to numeric values and displayed via a LCD. Further embodiments allow expression of the parameter as audible tones or as somatosensory vibratory stimuli on suitable frequency scales, or sending the signal to an application running on a mobile device using Android, Windows Mobile, or iOS, or to another sports monitoring device.

The selection of regions for electrode placement as disclosed in the current patent application has been made using extensive search algorithms over a big array of electrodes, preselected by plausibility assumptions. Notably, it does make a difference where to place the reference electrode. This can be demonstrated by simulations using the Matlab functions mscohere or cpsd. The reason why this makes a difference is that each EEG channel reflects an electrical potential between "sensing electrode" and "reference electrode". If the reference electrode is placed at a region which itself is influenced by brain (or other electrical) activity, the resulting signal depends on this activity. At different reference positions, thus, different signals are added/subtracted to/from the measurement. Phase coherence between the signals A and B resulting from these measurements is influenced by a signal C which is added to A and/or B, even it is added without scaling from both signals A and B.

WO 2009/087486 A2 propagates reference electrodes placed directly adjacent to the "sensing electrodes". This is part of a method to eliminate noise (noise is claimed to be essentially the same for sensing and reference electrode when these are located nearby and thus is cancelled out when determining the potential between the two electrodes). However, here is argued that relevant signal sources within the brain are picked up in similar way by nearby electrodes. Thus, not only noise but also signal is cancelled out with the method disclosed in that document. While this may result in low-noise signals, for the purpose of detecting the brains contribution to muscle fatigue, according to research, this is not useful.

Therefore, the inventors have identified a position for the reference electrode not adjacent to a sensing electrode as claimed in WO 2009/087486 A2. Here the position of electrodes is determined according to the process laid out in the paragraph reciting this prior art in view of a search of arrays of electrodes. Said document claims that there can be less reference electrodes than sensing electrodes which makes the system similar to ours insofar as there may be only one reference electrode. However, the document assumes the reference electrode to be positioned in close vicinity to a sensing electrode (e.g., within several millimetres, [0033]), which is not the case in the present application. Furthermore, WO 2009/087486 A2 discloses an apparatus which delivers a brain signal not indicative of muscle fatigue. Therefore the apparatus disclosed in said prior art cannot be used for the purpose described in the current application. Neither can it be used as an apparatus from which a person skilled in the art could directly derive a solution to the problem posed, since there is not a suitable position of the reference electrode available from said disclosure, nor is it clear which of the available sensing electrodes to use.

Further embodiments of the invention are laid down in the dependent claims.

These and other features of the present invention will be discussed in more detail below using a number of exemplary embodiments with reference to the accompanying drawings.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1    shows a block diagram of a system according to an embodiment of the invention;
Fig. 2    shows the positions of both cortical active electrodes and reference electrode on a 128-channel HCGSN v.1.0 using the system according to Fig. 1;
Fig. 3    shows the positions of both cortical active electrodes and reference electrode on a 10-10 Electrode Placement System using the system according to Fig. 1;
Fig. 4    shows an implementation of an electrode arrangement in a head system;
Fig. 5    shows a plurality of possible positions of both cortical active electrodes and reference electrode on a 10-10 Electrode Placement System using the system according to Fig. 1;

Fig. 6 shows a diagram of an output signal against time as a group average signal change, depicted as the double middle line ± SEM, from the beginning to the end of an exhaustive cycling exercise; and

Fig. 7 shows a statistical comparison of the signal during the first 15% (BEG) of cycling time with the last 15% (END) of cycling time.

DESCRIPTION OF PREFERRED EMBODIMENTS

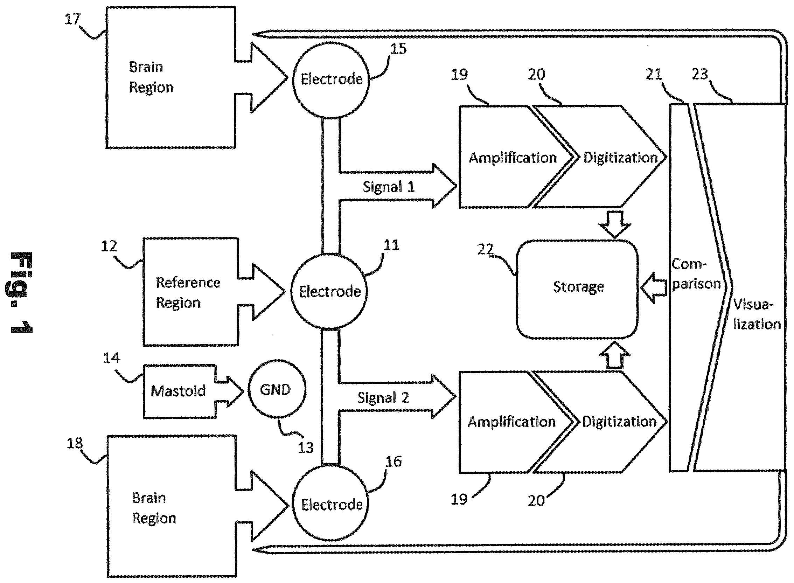

Referring to Fig. 1, there is shown an EEG biofeedback system in block diagram form in accordance with the present invention. A plurality of electrodes, including a cortical site reference electrode 11 are to be placed on a reference region 12 of the user, at least one ground electrode 13 placed on a mastoid 14 and at least two cortical site active electrodes 15, 16 placed over two distinct brain regions 17, 18.

Brain region 17 is selected such that the signal derived from it is influenced to a significant degree by neuronal activity in the primary motor cortex. This is achieved by placing the electrode 15 either within a region 31 on a user's scalp defined by the corner points 30-105-78-61, according to Fig. 2, or within a region 31 on a user's scalp defined by the corner points C1-C2-P2-P1, according to Fig. 3. Definition of the location of these points is given in the Geodesic Sensor Net Technical Manual SMAN200-GSNR-001, January 31, 2007, chapter 6, figure B-3 and in the American Clinical Neurophysiology Society, Guideline 5: Guidelines for Standard Electrode Position Nomenclature, 2006, figure 1, respectively. It is clear that also different reference coordinate systems can be chosen, since the electrode placements according to Fig. 2 and Fig. 3 will be simply transformed into such a different coordinate system.

Brain region 18 is selected such that the signal derived from it is influenced to a significant degree by neuronal activity in the insular cortex. This is achieved by placing the electrode 16 either within a region 32 on a user's scalp defined by the corner points 39-29-53-50, according to Fig. 2, or within a region 32 on a user's scalp defined by the corner points FT7-FC3-CP3-TP7, according to Fig. 3. Definition of the location of these points is also given in the Geodesic Sensor Net Technical Manual SMAN200-GSNR-001, January 31, 2007, chapter 6, figure B-3 and in the American Clinical Neurophysiology Society, Guideline 5: Guidelines for Standard Electrode Position Nomenclature, 2006, figure 1, respectively. The reference electrode 11 is placed on a position which is sufficiently independent from brain regions 17, 18 to evade signal cancellation while being sufficiently near to keep cable length short in order to evade picking up noise from surrounding electromagnetic fields.

In one specific embodiment, the reference electrode 11 is placed within a region 33 either on a user's scalp defined by the corner points 27-12-37-41, according to Fig. 2, or within a region 33 on a user's scalp defined by the corner points F5-F1-C1-C5, according to Fig. 3. Definition of the location of these points is again given in the Geodesic Sensor Net Technical Manual SMAN200-GSNR-001, January 31, 2007, chapter 6 and figure B-3 and in the American Clinical Neurophysiology Society, Guideline 5: Guidelines for Standard Electrode Position Nomenclature, 2006, figure 1, respectively. In further embodiments, further positions can be incorporated, thus increasing the number of channels.

Signal recording:

In a preferred embodiment, commercial electrodes are used for signal recording, ensuring good signal quality without the need for prior scalp preparation. Electrodes with salt water soaked pads (e.g. Emotiv EPOC, San Francisco, CA 94102, USA; Z-Elektroden, EEG bridge electrode, Schuler GmbH, Freiburg, Bielefeld) for deduction of an optimal signal during exhaustive activities under conditions of heat, sweating, and concussion. A multitude of such electrodes is freely available on the market.

To ensure that the recorded signal carries information from the relevant brain regions, in a preferred embodiment, electrodes are mounted on a headset (Fig. 4). Electrode positions will be set according to the requirements described above. The mounting system of the headset can be a stand-alone holding fixture or can be integrated in a helmet or a textile cap. Prior art allows for recording and display of brain signals during sports using an amplifier carried in a backpack (EEGoSports, ANT Neuro) or using electrodes at positions which cannot pick up brain signals relevant to muscle fatigue (EMOTIVE, https://emotiv.com). The current invention allows the use of the system during sports and displays online a brain activity dependent parameter relevant to muscle fatigue.

Signal processing:

Referring again to Fig. 1, signals are amplified using commercially available amplifiers 19 and converted to digital representation with 24 bit precision and a sampling rate of 250 Hz, using commercially available analog/digital converters 20. This approach represents standard steps in EEG signal processing. These steps result in at least two signals, which are further processed 21 using commercially available signal processors (DSP). Filtering, artefacts removal and processing of all channels as well as all further online-processes and calculations of all channels are done by programming code, known to the man skilled in the art. The code can be implemented in the DSP. After signal processing, the result signal is stored - together with the EEG raw data - on commercially available storage media for data storage 22.

Signal visualization and storage:

In a preferred embodiment, the result signal will be sent as graphic (e.g. red/orange/green LED) information to the visualization unit 23, a display mounted at the anterior end (e.g., visor) of the headset (Fig. 4). In other embodiments, the result signal may be transformed into an acoustic stimulus, vibratory stimulus, or any form of visual stimulus, or sent to an application running on a mobile device using Android, Windows Mobile, or iOS, or to another sports monitoring device.

Power supply:

Together with the electronics, the batteries for power supply are integrated in a small housing box 24 mounted at the back of the headset (Fig. 4). Commercially available batteries are used, which can be charged by connecting the device to a PC/Mac via USB, or by using a battery charger.

Data recording:

Data measurement and recording starts and stops by pressing a button on the electronics box 24 of the headset (Fig. 4). Another button serves to set event markers in order to define the beginning/end of a special training session and to analyse in real-time or after exercising how certain events (or strategies) affect the resulting signal.

Assembly of the device:

The display 23 as well as the electronics box 24 and the electrodes 11, 13, 15, 16 can be removed from the headset in order to allow for easy cleaning of both the headset and the hardware (Fig. 4). Thus, the preferred embodiment constitutes a self-contained mobile device capable of measuring, displaying and storing a brain physiological parameter representative of muscle fatigue.

Fig. 4 shows an implementation of an electrode arrangement in a head system with two perspective views from different directions. Within Fig. 4, the head system 10 has received the identical reference numerals for the different features as in Fig. 1 to 3. The head system 10 comprises a head band 40 surrounding the head 38 of the user. The head system 10 can comprise elements of a baseball cap as a long bill 41, i.e. the curved part sticking out in front, onto which underside the visualization unit 23 can be attached. The head band 40 can also be used to attach the housing box 24 for electronics and batteries.

The electrodes 11, 13, 15, 16 are provided each on an associated electrode patch 42, which is connected via a connection strip 43 to the head band 40. The connection strip 43 is a predefined strip with predetermined curved strip parts as 44 and 45 to predetermine the position of the corresponding electrode 11, 13, 15 or 16. The connection strips 43 are attached, optionally removably attached, to the head band 40, e.g. via a plastic snap-in or via Velcro connection. The connection with the associated electrode patch 42 is usually fix. The electrically conducting cable is not shown in the embodiment Fig. 4 or the cable is integrated into strip 43.

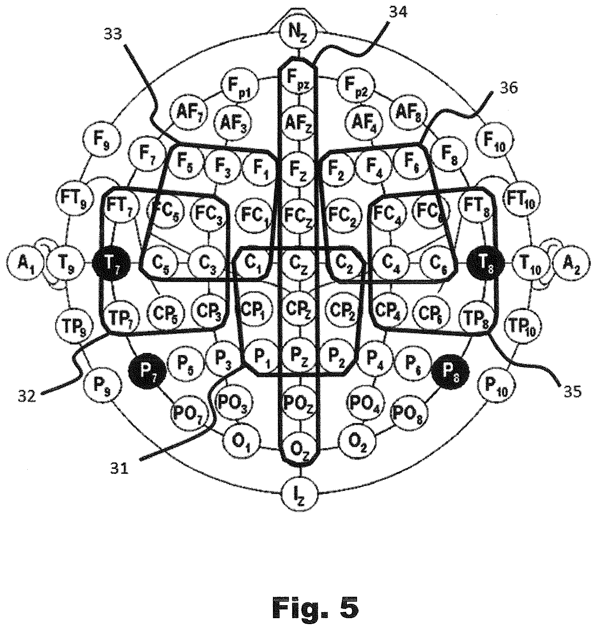

The predetermined orientation of the strips 43 and the band 40 on the head 38 of a user provides the correct positioning of the electrodes. It is possible to fix the headset 10 initially on a specimen head 38 with the distribution of the zones 31 (34), 32 and 33 as in Fig. 2 or Fig. 3 or (31) 34, 35, 36 as in Fig. 5. The reference numeral in parentheses relates to an alternative embodiment, since the reference electrode can be provided either on the middle line 34 or in the quadrangle 31. In another embodiment not shown in the drawings the electrodes are fixedly attached, optionally removably fixedly attached, on the inside of a helmet or hat at said positions in the areas 31 as well as 32 or 35 and 33 or 34 or 36 as in the drawings of the specification.

Fig. 5 shows a plurality of possible positions of both cortical active electrodes and reference electrode on a 10-10 Electrode Placement System using the system according to Fig. 1 (in addition to a ground electrode, not shown here). All identical or similar reference numerals relate to identical or similar features. Fig. 5 provides on the left side embodiments as shown in connection with Fig. 2 and Fig. 3 with the combination of electrode area 32 and 33 together with electrode area 31. Of course it is possible to provide the identical approach on the other side of the scalp in electrode areas 35 and 36, respectively, and together with electrode area 31. Electrode area 33 or 36 can be replaced by electrode area 34. Therefore, the electrode combinations from either side (one electrode in area 32 as well as one in area 33 or one electrode in area 35 as well as one in area 36) can be associated to a third electrode in area 31 or in area 34. In all cases a ground electrode is also provided. Said ground electrode can be positioned at the mastoid of the user but can also be provided elsewhere on the specimen's skin.

Fig. 6 shows a diagram of an output signal against time as a group average signal change, depicted as the

double middle line $\pm$ SEM, from the beginning to the end of an exhaustive cycling exercise. X-axis is scaled from 0 to 5000, interpolated such that each individual run was scaled to 5000 time units and the Y-axis scaled to arbitrary units, e.g. between 0 and 1, here between 0 and 10. It is clearly visible that the output signal increases over time due to the effort leading to exhaustion. The X-axis represents the time line. Here, the tests started for unexhausted users and was measured all the time until exhaustion.

Fig. 7 shows a statistical comparison of the signal during the first 15% (BEG) of cycling time with the last 15% (END) of cycling time. A significant signal increase (p=0.001, one tailed T-test), with a large effect size (Cohen's d = 1.17) is evident for and during an exhaustive cycling exercise. Y-axis scaled to arbitrary units. Usually, the fatigue ratio as shown on the Y-axis is calculated based on the two sensor signals in view of the reference electrode in view of the ground electrode. Said value of the output signal is often transformed into a value between 0 and 1.

LIST OF REFERENCE SIGNS

| | | | |
|---|---|---|---|
| 10 | head set | 32 | second region of scalp |
| 11 | reference electrode | 33 | region of scalp for reference |
| 12 | reference region (user) | | electrode |
| 13 | ground electrode | 34 | a further region of scalp for |
| 14 | mastoid (user) | | reference electrode |
| 15 | first cortical site electrode | 35 | a further second region of |
| 16 | second cortical site electrode | | scalp |
| 17 | first brain region | 36 | a further region of scalp for |
| 18 | second brain region | | reference electrode |
| 19 | amplifier | 38 | head |
| 20 | A/D converter | 40 | head band |
| 21 | processing step | 41 | bill or visor band |
| 22 | data storage | 42 | electrode patch |
| 23 | visualization unit | 43 | connection strip |
| 24 | housing box for electronics | 44 | strip portion |
| | and batteries | 45 | strip portion |
| 31 | first region of scalp | | |

**Claims**

1.   An apparatus for monitoring a brain signal, especially indicative of muscle fatigue, comprising at least three sensors (11, 15, 16) sensing neuronal activity, wherein one sensor (11) comprises a reference electrode and the other sensors (15, 16) comprise sensing electrodes, wherein the sensor (11) with the reference electrode serves as a common reference for the other sensors (15, 16), **characterized in that** the reference sensor (11) is placed within the quadrangle defined by the four positions [F5-F1-C1-C5] (33) or on the midline of the scalp (34), as defined by a corridor of up to 15 millimetres width between Fpz to Oz, the first sensing sensor (15) is placed within the quadrangle defined by the four positions [C1-C2-P2-P1] (31), and the second sensing sensor (16) is placed within the quadrangle defined by the four positions [FT7-FC3-CP3-TP7] (32), with the proviso that the reference electrode is not positioned on the same position as is one of the two sensing sensors (15, 16) and wherein the positions of the sensors delimiting the quadrangles are defined according to the 10-10 Electrode Placement System (American Clinical Neurophysiology Society, Guideline 5: Guidelines for Standard Electrode Position Nomenclature, 2006).

2.   An apparatus for monitoring a brain signal, especially indicative of muscle fatigue, comprising at least three sensors (11, 15, 16) sensing neuronal activity, wherein one sensor (11) comprises a reference electrode and the other sensors (15, 16) comprise sensing electrodes, wherein the sensor (11) with the reference electrode serves as a common reference for the other sensors (15, 16), **characterized in that** the reference sensor (11) is placed within the quadrangle defined by the four positions [F2-F6-C6-C2] (36) or on the midline of the scalp (34), as defined by a corridor of up to 15 millimetres width between Fpz to Oz, the first sensing sensor (15) is placed within the quadrangle defined by the four positions [C1-C2-P2-P1] (31), and the second sensing sensor (16) is placed within the quadrangle defined by the four positions [FC4-FT8-TP8-CP4] (35), with the proviso that the reference electrode is not positioned on the same position as is one of the two sensing sensors (15, 16) and wherein the positions of the sensors delimiting the quadrangles are defined according to the 10-10 Electrode Placement System (American Clinical Neurophysiology Society, Guideline 5: Guidelines for Standard Electrode Position Nomenclature, 2006).

3. The apparatus according to claim 1 or 2, further comprising a signal processor, receiving bioelectric sensor signals from the sensors, being connected with the reference electrode and optionally with one or more ground electrodes, and configured to perform a sequence of transformations on the bioelectric sensor signals received from the sensors quantifying an output signal $PS_{lagged}$, especially on a ratio scale, wherein the transformations include a.) a discrete Fourier transform of the signal time series from the first (15) as well as from the second (16) sensor; b.) a normalization of each of the two Fourier transforms to obtain phase information, insensitive to amplitude information; c.) the calculation of the complex valued cross spectra between the two normalized Fourier transforms; d.) a separation of the lagged components of phase synchronization from the instantaneous components by using the following formula: $PS_{lagged} = \sqrt{\dfrac{[\mathrm{Im}(S_{\check{x}\check{y}\omega})]^2}{1-[\mathrm{Re}(S_{\check{x}\check{y}\omega})]^2}}$ where $S_{\check{x}\check{y}\omega}$ denotes the cross spectra between the two normalized

Fourier transforms $\check{x}$ and $\check{y}$ at frequency $\omega$, Im denotes the imaginary part, and Re denotes the real part; and e.) providing the resulting value as said output signal $PS_{lagged}$ as indicative of supra-spinal muscle fatigue.

4. The apparatus according to any one of claims 1 to 3, wherein the one or more ground electrodes (13) are attached to either a left or right mastoid, a left or right earlobe, to another part of the specimen's skin or any combination of these.

5. The apparatus according to any one of claims 1 to 4, wherein the mounting system (40; 43) of the headset (10) can be a stand-alone holding fixture or can be integrated in a helmet, hat or a textile cap.

6. The apparatus of any one of claims 1 to 5, wherein the apparatus comprises a control unit which is configured to only sense information relating to neuronal signals containing information from a predetermined certain frequency range, especially from one, two or three elements of frequency ranges of the group of frequency ranges consisting of the frequency range between 7 and 13 Hz, of the frequency range between 3 and 7 Hz, of the frequency range between 13 and 20 Hz.

7. The apparatus of any one of claims 1 to 6, wherein the control unit is configured to detect said neuronal signal at a sampling rate of 250 Hz and with 24 bit precision.

8. The apparatus of any one of claims 1 to 7, further comprising storage means (22) for storing neuronal signals.

9. The apparatus of any one of claims 1 to 8, further comprising an output unit (23) to output the neuronal signal in real-time.

10. The apparatus of claim 9, wherein the output unit (23) is a display, a loudspeaker or a vibrator to be attached to the user or the cap, hat or helmet.

11. The apparatus of claim 10, wherein said neuronal signal is transported to a visual display unit mounted within a user's field of view, or to a visual display unit contained in a mobile device running on Android, Windows Mobile, or iOS, or to a visual display unit contained in another sports monitoring device.

12. The apparatus of any one of claims 1 to 11, wherein the reference sensor (11) is placed on the midline of the scalp (34), as defined by a corridor of up to 10 millimetres width between Fpz to Oz.

**Fig. 1**

**Fig. 2**

**Fig. 3**

# Fig. 4

**Fig. 5**

**Fig. 6**

**Fig. 7**

**EUROPEAN SEARCH REPORT**

Application Number

EP 16 20 7160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/087486 A2 (EMOTIV SYSTEMS PTY LTD [AU]; DELIC EMIR [AU]) 16 July 2009 (2009-07-16) | 1,2,4-12 | INV. A61B5/0476 A61B5/0478 A61B5/00 |
| Y | * paragraph [0027] * * paragraph [0029] - paragraph [0035]; figure 1 * * paragraph [0036] - paragraph [0037]; figure 2 * * paragraph [0038] * * paragraph [0053] * | 3 | |
| X | WO 2014/152806 A1 (ENCEPHALODYNAMICS INC [US]) 25 September 2014 (2014-09-25) * page 12, line 13 - line 18; figure 5 * * page 12, line 19 - page 13, line 16; figure 6 * | 1,2,4-11 | |
| Y,D | Roberto D Pascual-Marqui: "Instantaneous and lagged measurements of linear and nonlinear dependence between groups of multivariate time series: frequency decomposition", 9 November 2007 (2007-11-09), pages 1-18, XP055360199, https://arxiv.org/abs/0711.1455 Retrieved from the Internet: URL:https://arxiv.org/ftp/arxiv/papers/0711/0711.1455.pdf [retrieved on 2017-03-29] * 3. Measures of linear dependence (coherence-type) between two multivariate time series * * Equation 28 * * the whole document * | 3 | |

TECHNICAL FIELDS SEARCHED (IPC)

A61B

The present search report has been drawn up for all claims

| Place of search | Date of completion of the search | Examiner |
|---|---|---|
| The Hague | 12 May 2017 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 16 20 7160

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2017

| Patent document cited in search report | Publication date | Patent family member(s) | Publication date |
|---|---|---|---|
| WO 2009087486 A2 | 16-07-2009 | NONE | |
| WO 2014152806 A1 | 25-09-2014 | EP 2967387 A1<br>JP 2016530897 A<br>US 2016022165 A1<br>WO 2014152806 A1 | 20-01-2016<br>06-10-2016<br>28-01-2016<br>25-09-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4031883 A
- WO 2010147599 A1
- EP 0381090 A2
- US 2002120208 A1
- US 5267570 A
- WO 2013040642 A1
- KR 20030021585 A
- WO 2009087486 A2

### Non-patent literature cited in the description

- **BIGLAND-RITCHIE et al.** *Clin Sci Mol Med,* 1978, vol. 54, 609-14
- **TAYLOR et al.** *Clin Exp Pharmacol Physiol,* 2006, vol. 33, 400-405
- **HILTY et al.** *Eur J Neurosci,* 2011, vol. 34, 2035-2042
- **P. RASMUSSEN ; H. STIE ; L. NYBO ; B. NIELSEN.** Heat induced fatigue and changes of the EEG is not related to reduced perfusion of the brain during prolonged exercise in humans. *Journal of Thermal Biology,* October 2004, vol. 29 (7-8), ISSN 0306-4565, 731-737, http://dx.doi.org/10.1016/j.jtherbio.2004.08.047
- **J JOHNSTON ; M REARICK ; S SLOBOUNOV.** Movement-related cortical potentials associated with progressive muscle fatigue in a grasping task. *Clinical Neurophysiology,* January 2001, vol. 112 (1), ISSN 1388-2457, 68-77, http://dx.doi.org/!0.1016/S1388-2457(00)00452-1
- **LIU, J. Z. ; YAO, B. ; SIEMIONOW, V. ; SAHGAL, V. ; WANG, X. ; SUN, J. ; YUE, G. H.** Fatigue induces greater brain signal reduction during sustained than preparation phase of maximal voluntary contraction. *Brain research,* 2005, vol. 1057 (1), 113-126
- **JAIN, S. ; GOURAB, K. ; SCHINDLER-IVENS, S. ; SCHMIT, B. D.** EEG during pedalling: evidence for cortical control of locomotor tasks. *Clinical Neurophysiology,* 2013, vol. 124 (2), 379-390
- **A. A. ABDUL-LATIF ; I. COSIC ; D. K. KUMAR ; B. POLUS ; C. DA COSTA.** Power changes of EEG signals associated with muscle fatigue: the root mean square analysis of EEG bands. *Intelligent Sensors, Sensor Networks and Information Processing Conference, 2004. Proceedings of the 2004,* 2004, 531-534
- **HILTY, L. ; LANGER, N. ; PASCUAL-MARQUI, R. ; BOUTELLIER, U. ; LUTZ, K.** Fatigue-induced increase in intracortical communication between mid/anterior insular and motor cortex during cycling exercise. *European Journal of Neuroscience,* 2011, vol. 34, 2035-2042